# DEMANDE DE BREVET EUROPEEN

(11) **EP 0 554 167 A1**
(43) Date de publication de la demande: **04.08.1993**
(21) Numéro de dépôt: 93400193.4
(22) Date de dépôt: 27.01.1993
(51) Int. Cl.: C07C 69/743, A01N 53/00, C07C 255/39, C07D 207/333, C07D 213/647, C07D 277/24, C07D 307/45, C07C 61/40

(54) **Nouveaux dérivés de l'acide 2,2-diméthyl 3-/(2,2-difluorocyclopropylidène)méthyl/cyclopropane carboxylique, leur procédé de préparation de leur application comme pesticides**

(30) Priorité: 28.01.1992 FR 9200865
(71) Demandeur: ROUSSEL-UCLAF, F-75007 Paris (FR)
(72) Inventeur: Babin, Didier, F-78180 Montigny (FR); Benoit, Marc, F-13360 Roquevaire (FR); Demoute, Jean-Pierre, F-93360 Neuilly Plaisance (FR); Pilorge, Fabienne, F-77220 Tournan en Brie (FR); Reinier, Nicole, F-13009 Marseille (FR)
(74) Mandataire: Tonnellier, Marie-José

(57) **Abrégé**

L'invention a pour objet sous toutes leurs formes stéréo-isomères possibles, ainsi que leurs mélanges les composés de formule (I) : 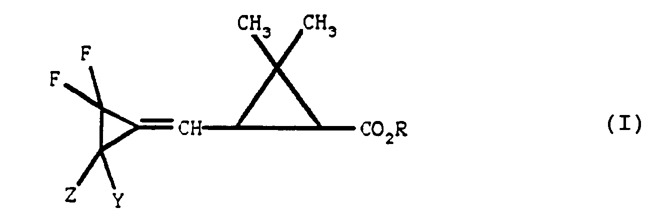
dans lesquels :
- Y et Z, identiques ou différents l'un de l'autre, représentent un atome d'hydrogène, un atome d'halogène, un radical CF₃, un radical alkyle renfermant jusqu'à 8 atomes de carbone, un radical O-alkyle, renfermant jusqu'à 8 atomes de carbone, ou un radical S-alkyle renfermant jusqu'à 8 atomes de carbone, ou un radical aryle, O-aryle ou S-aryle renfermant jusqu'à 14 atomes de carbone éventuellement substitué par un ou plusieurs atomes d'halogène, un ou plusieurs radicaux hydroxyle estérifiés ou éthérifiés, un ou plusieurs radicaux CF₃, un ou plusieurs radicaux alkyle renfermant jusqu'à 8 atomes de carbone,
- R représente un radical alkyle renfermant jusqu'à 8 atomes de carbone, ou le reste d'un alcool utilisé dans la synthèse des esters pyréthrinoïdes,

sous toutes leurs formes stéréoisomères possibles ainsi que leurs mélanges.

Les composés de formule (I) présentent d'intéressantes propriétés pesticides.

## Description

La présente invention concerne de nouveaux dérivés de l'acide 2,2-diméthyl 3-[(2,2-difluorocyclopropylidène) méthyl] cyclopropane carboxylique, leur procédé de préparation et leur application comme pesticides.

L'invention a pour objet sous toutes leurs formes stéréoisomères possibles, ainsi que leurs mélanges les composés de formule (I) : 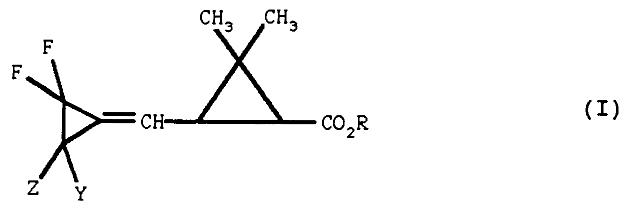
dans lesquels :
- Y et Z, identiques ou différents l'un de l'autre, représentent un atome d'hydrogène, un atome d'halogène, un radical CF₃, un radical alkyle renfermant jusqu'à 8 atomes de carbone, un radical O-alkyle, renfermant jusqu'à 8 atomes de carbone, ou un radical S-alkyle renfermant jusqu'à 8 atomes de carbone, ou un radical aryle, O-aryle ou S-aryle renfermant jusqu'à 14 atomes de carbone éventuellement substitué par un ou plusieurs atomes d'halogène, un ou plusieurs radicaux hydroxyle estérifiés ou éthérifiés, un ou plusieurs radicaux CF₃, un ou plusieurs radicaux alkyle renfermant jusqu'à 8 atomes de carbone,
- R représente un radical alkyle renfermant jusqu'à 8 atomes de carbone, ou le reste d'un alcool utilisé dans la synthèse des esters pyréthrinoïdes,

sous toutes leurs formes stéréoisomères possibles ainsi que leurs mélanges.

La géométrie de la double liaison peut être E ou Z ou un mélange E + Z. La copule 2,2-diméthyl cyclopropanique peut être de structure 1R cis ou 1R trans ou un mélange 1R cis, 1R trans. Lorsque Z ne représente pas un atome d'hydrogène, la copule 2,2-difluorocyclopropanique peut être de structure 1R cis ou 1R trans ou un mélange 1R cis, 1R trans.

L'invention a pour objet chacun des isomères possibles, ainsi que les mélanges de ces isomères.

Par radical alkyle, on entend de préférence un radical alkyle linéaire, ramifié ou cyclique, saturé ou insaturé, par exemple un radical méthyle, éthyle, n-propyle, isopropyle, n-butyle, isobutyle, tert-butyle, d'un radical cyclopropyle, cyclobutyle, cyclopentyle ou cyclohexyle, d'un radical vinyle ou 1,1-diméthylallyle ou d'un radical acétylénique comme par exemple le radical éthynyle ou propynyle.

Lorsque R représente un radical alkylidène substitué par un atome d'halogène, il peut s'agir du radical CF₂=CH-.

Par radical aryle, on entend de préférence un radical phényle ou napthyle.

Par radical hydroxyle estérifié ou éthérifié, on entend de préférence un radical OCH₃, OC₂H₅, OC₃H₇, OCOCH₃, OCOC₆H₅.

L'invention a plus particulièrement pour objet les composés de formule (I) dans lesquels Y représente un atome d'hydrogène.

Parmi ceux-ci l'invention a plus particulièrement pour objet les composés de formule (I) dans lesquels Z représente un atome d'hydrogène.

L'invention a tout particulièrement pour objet les composés de formule (I) dans lesquels la copule 2,2-diméthyl cyclopropanique est de structure 1R cis ainsi que les composés de formule (I) dans lesquels la géométrie de la double liaison est E.

L'invention a notamment pour objet les composés de formule (I) dans lesquels R représente :
- soit un radical alkyle, renfermant jusqu'à 8 atomes de carbone,
- soit un radical benzyle éventuellement substitué par un ou plusieurs radicaux choisis dans le groupe constitué par les radicaux alkyles comportant de 1 à 4 atomes de carbone, éventuellement substitués par un ou plusieurs atomes d'halogène par exemple les radicaux CF₃, les radicaux alkyloxyles comportant de 1 à 4 atomes de carbone éventuellement substitués par un ou plusieurs atomes d'halogène par exemple les radicaux OCHF₂, les radicaux alcényles comportant de 2 à 6 atomes de carbone, les radicaux alcényloxy comportant de 2 à 6 atomes de carbone, les radicaux alcadiényles comportant de 4 à 8 atomes de carbone, le radical méthylènedioxy et les atomes d'halogène,
- soit un groupement : 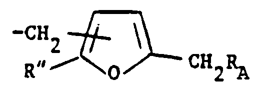 dans lequel le substituant R'' représente un atome d'hydrogène ou un radical méthyle et le substituant R_{A} un aryle monocyclique ou un groupement -C≡CH et notamment un groupement 5-benzyl 3-furyl méthyle,
- soit un groupement : 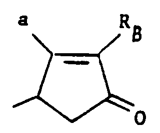 dans lequel a représente un atome d'hydrogène ou un radical méthyle et R_{B} représente un radical organique aliphatique comportant de 2 à 6 atomes de carbone et une ou plusieurs insaturations carbone-carbone et notamment le radical -CH₂-CH=CH₂, -CH₂-CH=CH-CH₃, -CH₂-CH=CH-CH=CH₂, -CH₂-CH=CH-CH₂-CH₃, -CH₂-C≡CH,
- soit un groupement : 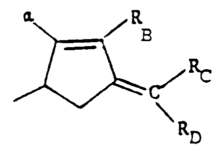 dans lequel a et R_{B} conservent la même signification que précédemment, R_{C} et R_{D}, identiques ou différents, représentent un atome d'hydrogène, un atome d'halogène, un radical alkyle renfermant de 1 à 6 atomes de carbone, un radical aryle comportant de 6 à 10 atomes de carbone, un groupement alkyloxycarbonyle comportant de 2 à 5 atomes de carbone, ou un groupement cyano,
- soit un groupement : 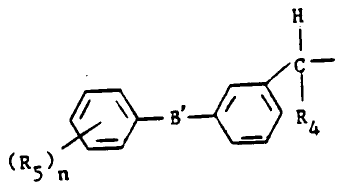 dans lequel B' représente un atome d'oxygène ou de soufre, un groupement 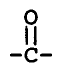 ou -CH₂ ou un groupement sulfoxyde ou un groupement sulfone et R₄ représente un atome d'hydrogène, un radical -C≡N, un radical méthyle, un radical -CONH₂, un radical -CSNH₂ ou un radical -C≡CH, R₅ représente un atome d'hydrogène, un atome d'halogène ou un radical méthyle et n représente un nombre égal à 0,1 ou 2, et notamment le groupement 3-phénoxy benzyle, alpha-cyano 3-phénoxy benzyle, alpha-éthynyl 3-phénoxy benzyle, 3-benzoyl benzyle, 1-(3-phénoxy phényl) éthyle ou alpha-thioamido 3-phénoxy benzyle,
- soit un groupement : 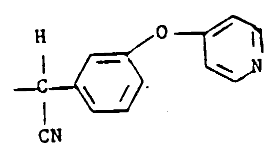
- soit un groupement : 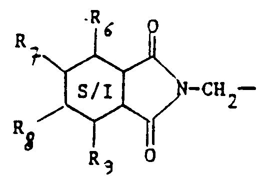 dans lequel les substituants R₆, R₇, R₈, R₉ représentent un atome d'hydrogène, un atome de chlore, ou un radical méthyle et dans lequel S/I symbolise un cycle aromatique ou un cycle analogue dihydro ou tétrahydro,
- soit un groupement : 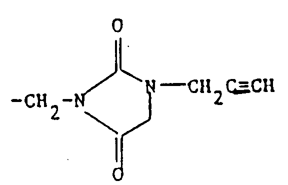
- soit un groupement : 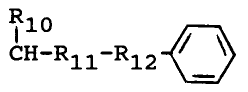 dans lequel R₁₀ représente un atome d'hydrogène ou un radical CN, R₁₂ représente un radical -CH₂- ou un atome d'oxygène, R₁₁ représente un radical thiazolyle ou thiadiazolyle dont la liaison avec 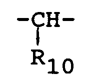 peut se trouver à l'une quelconque des positions disponibles, R₁₂ étant lié à R₁₁ par l'atome de carbone compris entre l'atome de soufre et un atome d'azote,
- soit un groupement : 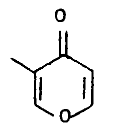
- soit un groupement : 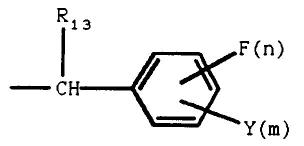 dans lequel R₁₃ représente un atome d'hydrogène, un radical CH₃, C≡N ou C≡CH,
   n représente un nombre entier pouvant varier de 1 à 5,
   m représente le nombre 5-n et,
- Y représente un atome d'hydrogène, un atome d'halogène, un radical CH₂-C≡N, un radical hydroxy, un radical alkyle linéaire, ramifié, saturé ou insaturé éventuellement substitué renfermant jusqu'à 8 atomes de carbone un radical nitrile, un radical 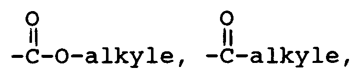 -(CH₂)m' O-alkyle, -(CH₂)m' S-alkyle, -(CH₂)m' N-(alkyle)₂, dans lequel m' représente le nombre 0, 1, 2, 3 ou 4 et renfermant jusqu'à 12 atomes de carbone, un radical Si (alkyle)₃, alkyle représente un radical alkyle linéaire ou ramifié, saturé ou insaturé, éventuellement substitué renfermant jusqu'à 8 atomes de carbone, un radical O-aryle ou -(CH₂)m'-aryle, aryle représentant un radical aryle renfermant jusqu'à 14 atomes de carbone, le ou les substituants Y identiques ou différents, pouvant être en position quelconque sur le noyau phényle,
- soit un groupement : 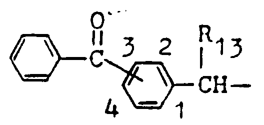 dans lequel R₁₃ est défini comme ci-dessus, et le radical benzoyle est lié aux positions 3 ou 4 du phényle,
- soit un groupement : 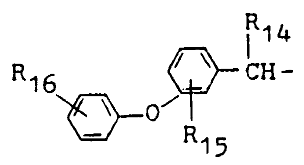 dans lequel R₁₄ représente un atome d'hydrogène, un radical méthyle, éthynyle ou cyano et R₁₅ et R₁₆, différents, représentent un atome d'hydrogène, de fluor ou de brome,
- soit un groupement : 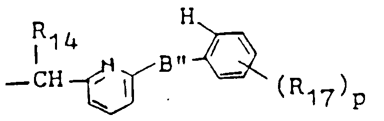 dans lequel R₁₄ est défini comme ci-dessus, chacun des R₁₇ représente indépendamment un atome d'hydrogène, un groupement alkyle renfermant de 1 à 4 atomes de carbone, alcoxy renfermant de 1 à 4 atomes de carbone, alkylthio renfermant de 1 à 4 atomes de carbone, alkylsulfonyl renfermant de 1 à 4 atomes de carbone, trifluorométhyl, 3,4-méthylènedioxy, chloro, fluoro ou bromo, p représente un nombre égal à 0,1 ou 2 et B'' représente un atome d'oxygène ou un atome de soufre,
- soit un groupement : 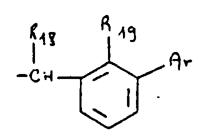 dans lequel R₁₈ représente un atome d'hydrogène, un radical méthyle, éthynyle ou cyano et R₁₉, différent, représente un atome d'hydrogène, de fluor ou de brome et Ar représente un radical aryle renfermant jusqu'à 14 atomes de carbone,
- soit un groupement 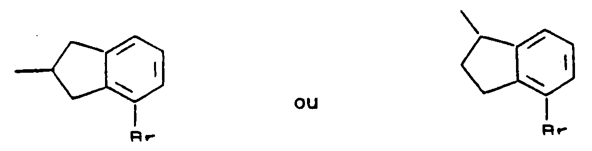 dans lequel Ar est défini comme précédemment,
- soit un groupement : 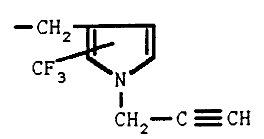 dans lequel le radical CF₃ est en position quelconque sur le noyau pyrrolique,
- soit un groupement 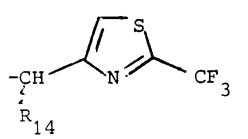 dans lequel R₁₄ est défini comme ci-dessus,
- soit un groupement 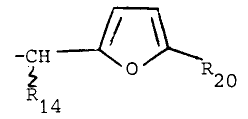 dans lequel R₁₄ est défini comme ci-dessus et R₂₀ représente un radical alkyle comportant de 1 à 4 atomes de carbone éventuellement substitué par un ou plusieurs atomes d'halogène.

Parmi les composés préférés de l'invention, on peut citer :
- les composés de formule (I) dans lesquels R représente un radical : 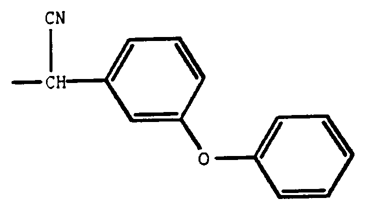
- les composés de formule (I) dans lesquels R représente un radical : 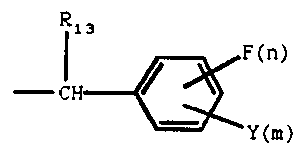 dans lequel R₁₃, m, n et Y sont définis comme précédemment,
- les composés de formule (I) dans lesquels R représente un radical : 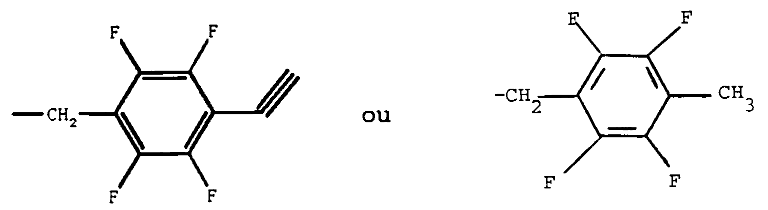
- les composés de formule (I) dans lesquels R représente un radical : 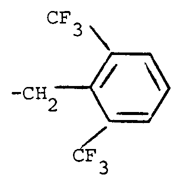
- les composés de formule (I) dans lesquels R représente un radical 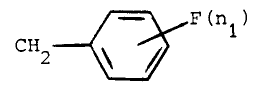 dans lequel n₁ = 3, 4 ou 5,
- les composés de formule (I) dans lesquels R représente un radical 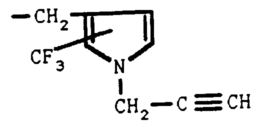
- les composés de formule (I) dans lesquels R représente un radical 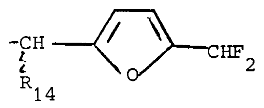 dans lequel R₁₄ représente un atome d'hydrogène ou un radical éthynyle,
- les composés de formule (I) dans lesquels R représente un radical

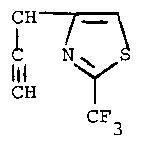

L'invention a notamment pour objet les composés dont la préparation est donnée ci-après dans la partie expérimentale. parmi les composés préférés de l'invention, on peut citer les composés des exemples 2, 4, 6, 7, 8, 11, 12, 15, 16, 18 et 22.

Les composés de formule (I) présentent d'intéressantes propriétés qui permettent leur utilisation dans la lutte contre les parasites. Il peut s'agir par exemple de la lutte contre les parasites des végétaux, qu'il s'agisse des parasites du sol ou des parties aériennes, les parasites des locaux et les parasites des animaux à sang chaud.

C'est ainsi que l'on peut utiliser les produits de l'invention pour lutter contre les insectes, les nématodes et les acariens parasites des végétaux et des animaux.

L'invention a notamment pour objet l'application des composés de formule (I) à la lutte contre les parasites des végétaux, les parasites des locaux et les parasites des animaux à sang chaud.

Les produits de formule (I) peuvent aussi être utilisés pour lutter contre les insectes et autres parasites du sol, par exemple les coléoptères, comme DIABROTICA, les taupins et les vers blancs, les myriapodes comme les scutigérelles et les blaniules, et les diptères comme les cécydomies et les lépidoptères comme les noctuelles terricoles.

Ils sont utilisés à des doses comprises entre 10 g et 300 g de matière active à l'hectare.

Les produits de formule (I) peuvent également être utilisés pour lutter contre les insectes dans les locaux, pour lutter notamment contre les mouches, les moustiques et les blattes.

Les produits de formule (I) sont de plus photostables et sont peu toxiques pour les mammifères.

L'ensemble de ces propriétés fait des produits de formule (I) des produits qui correspondent parfaitement aux exigences de l'industrie agrochimique moderne : ils permettent de protéger les récoltes tout en préservant l'environnement.

Les produits de formule (I) peuvent aussi être utilisés pour lutter contre les acariens et les nématodes parasites des végétaux.

Les composés de formule (I) peuvent encore être utilisés pour lutter contre les acariens parasites des animaux, pour lutter par exemple contre les tiques et notamment les tiques du genre de Boophilus, ceux du genre Hyalomnia, ceux du genre Amblyomnia et ceux du genre Rhipicephalus ou pour lutter contre toutes sortes de gales et notamment la gale sarcoptique, la gale psoroptique et la gale chorioptique.

L'invention a donc également pour objet les compositions destinées à la lutte contre les parasites des animaux à sang chaud, les parasites des locaux et des végétaux, caractérisées en ce qu'elles renferment au moins l'un des produits de formule (I) définis ci-dessus et notamment les produits des exemples 2, 4, 6, 7, 8, 11, 12, 15, 16, 18 et 22.

L'invention a notamment pour objet les compositions insecticides renfermant comme principe actif au moins l'un des produits définis ci-dessus.

Ces compositions sont préparées selon les procédés usuels de l'industrie agrochimique ou de l'industrie vétérinaire ou de l'industrie des produits destinés à la nutrition animale.

Dans ces compositions destinées à l'usage agricole et à l'usage dans les locaux, la ou les matières actives peuvent être additionnées éventuellement d'un ou plusieurs autres agents pesticides. Ces compositions peuvent se présenter sous forme de poudres, granulés, suspensions, émulsions, solutions, solutions pour aérosols, bandes combustibles, appâts ou autres préparations employées classiquement pour l'utilisation de ce genre de composés.

Outre le principe actif, ces compositions contiennent, en général, un véhicule et/ou un agent tensio-actif, non ionique, assurant, en outre, une dispersion uniforme des substances constitutives du mélange. Le véhicule utilisé peut être un liquide, tel que l'eau, l'alcool, les hydrocarbures ou autres solvants organiques, une huile minérale, animale ou végétale, une poudre telle que le talc, les argiles, les silicates, le kieselguhr ou un solide combustible.

Les compositions insecticides selon l'invention contiennent de préférence de 0,005 % à 10 % en poids de matière active.

Selon un mode opératoire avantageux, pour un usage dans les locaux, les compositions selon l'invention sont utilisées sous forme de compositions fumigantes.

Les compositions selon l'invention peuvent alors être avantageusement constituées, pour la partie non active, d'un serpentin insecticide (ou coil) combustible, ou encore d'un substrat fibreux incombustible. Dans ce dernier cas, le fumigant obtenu après incorporation de la matière active est placé sur un appareil chauffant tel qu'un émanateur électrique.

Dans le cas où l'on utilise un serpentin insecticide, le support inerte peut être, par exemple, composé de marc de pyrèthre, poudre de Tabu (ou poudre de feuilles Machilus Thumbergii), poudre de tige de pyrèthre, poudre de feuille de cèdre, poudre de bois (telle que de la sciure de pin) amidon et poudre de coque de noix de coco.

La dose de matière active peut alors être, par exemple, de 0,03 à 1 % en poids.

Dans le cas où l'on utilise un support fibreux incombustible, la dose de matière active peut alors être, par exemple, de 0,03 à 95 % en poids.

Les compositions selon l'invention pour un usage dans les locaux peuvent aussi être obtenues en préparant une huile pulvérisable à base de principe actif, cette huile imbibant la mèche d'une lampe et étant alors soumise à la combustion.

La concentration du principe actif incorporé à l'huile est, de préférence, de 0,03 à 95 % en poids.

L'invention a également pour objet les compositions acaricides renfermant comme principe actif au moins un des produits de formule (I) définie ci-dessus.

Les compositions insecticides selon l'invention, comme les compositions acaricides et nématicides, peuvent être additionnées éventuellement d'un ou plusieurs autres agents pesticides. Les compositions acaricides et nématicides peuvent se présenter notamment sous forme de poudre, granulés, suspensions, émulsions, solutions.

Pour l'usage acaricide, on utilise de préférence des poudres mouillables, pour pulvérisation foliaire, contenant de 1 à 80 % ou des liquides pour pulvérisation foliaire contenant de 1 à 500 g/l de principe actif. On peut également employer des poudres pour poudrages foliaires contenant de 0,05 à 3 % de matière active.

Pour l'usage nématicide, on utilise de préférence des liquides pour traitement des sols contenant de 300 à 500 g/l de principe actif.

Les composés acaricides et nématicides selon l'invention sont utilisés, de préférence, à des doses comprises entre 1 et 100 g de matière active à l'hectare.

Pour exalter l'activité biologique des produits de l'invention on peut les additionner à des synergistes classiques utilisés en pareil cas tel que le 1-(2,5,8-trioxadodécyl) 2-propyl 4,5-méthylènedioxy benzène (ou butoxyde de pipéronyle) ou la N-(2-éthyl heptyl) bicyclo /2,2-1/ 5- heptène-2,3-dicarboximide, ou le pipéronyl-bis-2-(2'-n-butoxy éthoxy) éthylacétal (ou tropital).

Les composés de formule (I) présentent une excellente tolérance générale, et l'invention a donc également pour objet les produits de formule (I), pour lutter notamment contre les affections créées par les tiques et les gales chez l'homme et l'animal.

Les produits de l'invention sont notamment utilisés pour lutter contre les poux à titre préventif ou curatif et pour lutter contre la gale.

Les produits de l'invention peuvent être administrés par voie externe, par vaporisation, par shampooing, par bain ou badigeonnage.

Les produits de l'invention à usage vétérinaire peuvent être également administrés par badigeonnage de l'épine dorsale selon la méthode dite méthode "pour-on".

On peut indiquer également que les produits de l'invention peuvent être utilisés comme biocides ou comme régulateurs de croissance.

L'invention a également pour objet les associations douées d'activité insecticide, acaricide ou nématicide, caractérisées en ce qu'elles contiennent comme matière active, d'une part un au moins des composés de formule générale (I), et d'autre part, un au moins des esters pyréthrinoïdes choisis dans le groupe constitué par les esters d'alléthrolone, d'alcool 3,4,5,6-tétrahydrophtalimido méthylique, d'alcool 5-benzyl 3-furyl méthylique, d'alcool 3-phénoxy benzylique et d'alcool alpha-cyano 3-phénoxy benzylique des acides chrysanthémiques, par les esters d'alcool 5-benzyl 3-furyl méthylique des acides 2,2-diméthyl 3-(2-oxo 3-tétrahydrothiophénylidène méthyl) cyclopropane-1-carboxyliques, par les esters d'alcool 3-phénoxy benzylique et d'alcool alpha-cyano 3-phénoxy benzylique des acides 2,2-diméthyl 3-(2,2-dichlorovinyl) cyclopropane-1-carboxyliques, par les esters d'alcool alpha-cyano 3-phénoxy benzylique d'acides 2,2-diméthyl 3-(2,2-dibromovinyl) cyclopropane-1-carboxyliques, par les esters d'alcool 3-phénoxy benzylique des acides 2-parachlorophényl 2-isopropyl acétiques, par les esters d'alléthrolone, d'alcool 3,4,5,6-tétrahydrophtalimidométhylique, d'alcool 5-benzyl 3-furyl méthylique, d'alcool 3-phénoxy benzylique et d'alcools alpha-cyano 3-phénoxy benzyliques des acides 2,2-diméthyl 3-(1,2,2,2-tétrahaloéthyl) cyclopropane-1-carboxyliques, dans lesquels "halo" représente un atome de fluor, de chlore ou de brome, étant entendu que les composés (I) peuvent exister sous toutes leurs formes stéréoisomères possibles de même que les copules acides et alcools des esters pyréthrinoïdes ci-dessus.

L'invention a également pour objet un procédé de préparation des composés de formule (I) caractérisé en ce que l'on soumet un acide de formule (II) : 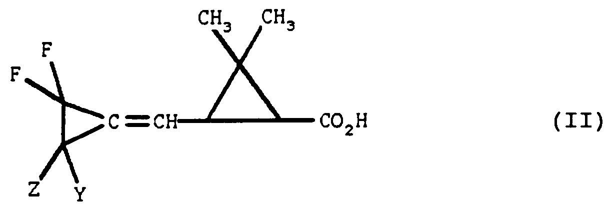
dans lequel Y et Z sont définis comme précédemment, ou un dérivé fonctionnel de cet acide, à l'action d'un alcool de formule (III) :

ROH (III)

R étant défini comme précédemment, pour obtenir le composé de formule (I) correspondant.

L'estérification de l'acide de formule (II) avec l'alcool de formule (III) peut être effectuée en présence d'un agent déshydratant, d'une base tertiaire telle que la pyridine.

Cette estérification peut être effectuée avantageusement en présence d'un mélange de dicyclohexylcarbodiimide et de pyridine ou de 4-diméthyl aminopyridine.

L'estérification peut également être réalisée en faisant réagir le chlorure de l'acide II avec l'alcool III ou sur un dérivé organométallique de cet alcool ou par transestérification à partir d'esters de l'acide de formule (II) notamment à partir de l'acétate.

Les produits de formule (III) sont des produits connus d'une façon générale. Certains produits sont nouveaux et leur préparation est donnée ci-après dans la partie expérimentale.

Les produits de formule (II) sont des produits nouveaux.

L'invention a également pour objet les acides de formule (II) : 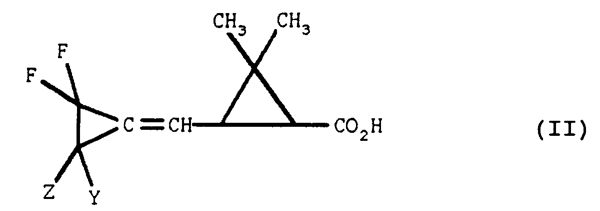
dans laquelle Y et Z sont définis comme précédemment.

L'invention a tout naturellement plus particulièrement pour objet les acides de formule (II) dont la préparation est donnée ci-après dans la partie expérimentale.

La préparation des acides de formule (II) peut être schématisée comme suit :

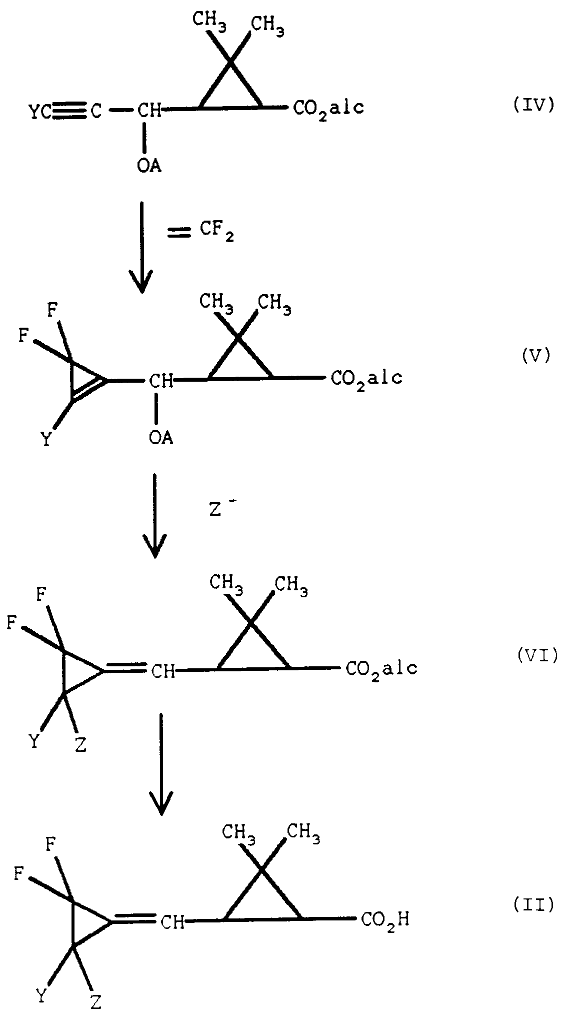

Dans les produits mis en oeuvre pour préparer les acides de formule (II), A représente le reste d'un acide carboxylique renfermant jusqu'à 8 atomes de carbone éventuellement substitué par un ou plusieurs atomes d'halogène par exemple l'acide acétique ou trifluoroacétique ou le reste d'un acide sulfonique par exemple le reste de l'acide mésylique ou tosylique.

Alc représente de préférence un radical alkyle renfermant jusqu'à 8 atomes de carbone.

Dans un mode de réalisation préféré de préparation des acides de formule (II), on fait réagir sur un composé de formule (IV) un composé générateur de difluorocarbène "in situ" comme par exemple ClCF₂CO₂Na, CF₂Br₂, CF₂ClH, CF₂Cl₂ pour obtenir le composé de formule (V) que l'on soumet à l'action d'un agent de transposition allylique. C'est-à-dire que lorsque l'on veut préparer des acides de formule (II), dans lesquels Z représente un atome d'hydrogène, on fait réagir un agent de réduction comme (φ₃PCuH)₆ ou φ₃KBH ou NaBH₄ en présence d'un catalyseur. Lorsque l'on veut préparer des acides de formule (II) dans lesquels Z ne représente pas un atome d'hydrogène, on fait réagir comme agent de transposition allylique, un générateur d'ion Z⁻ par exemple un générateur d'ion Hal⁻, CF₃⁻, alc⁻, Oalc⁻ ou Salc⁻.

On obtient les produits de formule (V) sous forme d'un mélange de stéréoisomères E et Z, que l'on sépare si désiré par chromatographie.

La transformation de l'ester (VI) en acide (II) a lieu selon les procédés classiques, par exemple action de l'acide trifluoroacétique. On peut si désiré séparer les mélanges d'acides E + Z en acides E et en acide Z par chromatographie.

Les composés de formule (IV) sont des produits connus d'une façon générale, ils peuvent être préparés selon le procédé décrit dans le brevet européen 0105006.

Le [1R-[1alpha, 3alpha(R)]] 2,2-diméthyl 3-(1-acétoxy propynyl) cyclopropane carboxylate de (1,1-diméthyl) éthyle est un produit nouveau et sa préparation est donnée ci-après dans la partie expérimentale. L'invention a donc également pour objet ce produit, à titre de produit industriel nouveau.

Les exemples suivants illustrent l'invention sans toutefois la limiter.

### EXEMPLE 1 : [1R-[1alpha, 3alpha(Z)]] 2,2-diméthyl 3-[(2,2-difluorocyclopropylidène) méthyl] cyclopropane carboxylate de 2,3,5,6-tétrafluoro phényl méthyle

On introduit une solution renfermant 280 mg de dicyclohexylcarbodiimide, 5,5 mg de 4-diméthylaminopyridine et 2 cm³ de chlorure de méthylène dans une solution renfermant 275 mg de l'isomère Z de l'acide dont la préparation est donnée ci-après (préparation 1) 245 mg d'alcool 2,3,5,6-tétrafluorobenzylique et 4 cm³ de chlorure de méthylène. On maintient l'agitation pendant 6 heures, filtre, rince à l'éther isopropylique et amène à sec. On obtient un produit que l'on chromatographie sur silice en éluant avec le mélange hexane-éther isopropylique (95-5). On obtient 240 mg du produit recherché. rf = 0,15 (hexane-éther isopropylique 95-5).

### EXEMPLE 2 : [1R-[1alpha, 3alpha(E)]] 2,2-diméthyl 3-[(2,2-difluorocyclopropylidène) méthyl] cyclopropane carboxylate de (2,3,5,6-tétrafluoro phényl) méthyle

En opérant comme à l'exemple 1, à partir de 420 mg de l'isomère E de l'acide obtenu à la préparation 1 et de 388 mg de l'alcool 2,3,5,6-tétrafluoro benzylique, on obtient 360 mg du produit recherché. rf = 0,15 (hexane-chlorure de méthylène 8-2). F = 61,5°C.

### EXEMPLE 3 : [1R-[1alpha, 3alpha(Z)]] 2,2-diméthyl 3-[(2,2-difluorocyclopropylidène) méthyl] cyclopropane carboxylate de (2,3,5,6-tétrafluoro 4-propynyl phényl) méthyle

En opérant comme à l'exemple 1, à partir de 275 mg de l'isomère Z de l'acide obtenu à la préparation 1 et de 300 mg d'alcool 2,3,5,6-tétrafluoro 4-propynyl benzylique, on obtient 400 mg de produit recherché. F = 70,7°C.

### EXEMPLE 4 : [1R-[1alpha, 3alpha(E)]] 2,2-diméthyl 3-[(2,2-difluorocyclopropylidène) méthyl] cyclopropane carboxylate de (2,3,5,6-tétrafluoro 4-propynyl phényl) méthyle

En opérant comme à l'exemple 1, à partir de 420 mg de l'isomère E de l'acide obtenu à la préparation 1 et 450 mg de l'alcool 2,3,5,6-tétrafluoro 4-propynyl benzylique, on obtient 470 mg de produit recherché. rf = 0,1 (hexane-chlorure de méthylène 8-2).

### EXEMPLE 5 : [1R-[1alpha, 3alpha(E,Z)]] 2,2-diméthyl 3-[(2,2-difluorocyclopropylidène) méthyl] cyclopropane carboxylate de [1-(2-propynyl) 2-(trifluoro méthyl) 1H-pyrrol 3-yl] méthyle

En opérant comme à l'exemple 1, à partir de 200 mg du mélange d'isomère E,Z de l'acide obtenu à la préparation 1 et 200 mg de l'alcool [1-(2-propynyl) 2-(trifluorométhyl) 1H-pyrrol 3-yl] méthylique, on obtient 240 mg de produit recherché. rf = 0,10 (hexane-éther isopropylique 9-1).

### EXEMPLE 6 : [1R-[1alpha(s), 3alpha(E,Z)]] 2,2-diméthyl 3-[(2,2-difluorocyclopropylidène) méthyl] cyclopropane carboxylate de 1-cyano (3-phénoxyphényl) méthyle

En opérant comme à l'exemple 1, à partir du mélange d'isomères E,Z de l'acide obtenu à la préparation 1 et de l'alcool 1-cyano 3-phénoxy benzylique, on obtient le produit recherché. rf = 0,08 (hexane-éther isopropylique 9-1).

En opérant comme à l'exemple 1, on a obtenu les produits recherché à partir de l'acide et de l'alcool appropriés.

### EXEMPLE 7 : [1R-[1alpha, 3alpha(E)]] 2,2-diméthyl 3-[(2,2-difluorocyclopropylidène) méthyl] cyclopropane carboxylate de (pentafluorophényl) méthyle

rf = 0,2 (hexane-éther isopropylique 98-2)

### EXEMPLE 8 : [1R-[1alpha, 3alpha(E)]] 2,2-diméthyl 3-[(2,2-difluorocyclopropylidène) méthyl] cyclopropane carboxylate de (4-méthyl 2,3,5,6-tétrafluorophényl) méthyle

rf = 0,1 (hexane-éther isopropylique 98-2)

### EXEMPLE 9 : [1R-[1alpha, 3alpha(E)]] 2,2-diméthyl 3-[(2,2-difluorocyclopropylidène) méthyl] cyclopropane carboxylate de (2,6-difluorophényl) méthyle

rf = 0,15 (pentane-chlorure de méthylène 8-2)

### EXEMPLE 10 : [1R-[1alpha, 3alpha(E)]] 2,2-diméthyl 3-[(2,2-difluorocyclopropylidène) méthyl] cyclopropane carboxylate de (3-difluorométhoxyphényl) méthyle

L'alcool 3-difluorométhoxy benzylique utilisé au départ a été préparé comme indiqué dans le brevet DE 2831193A1.
rf = 0,3 (hexane-éther isopropylique 9-1)

### EXEMPLE 11 : [1R-[1alpha, 3alpha(E)]] 2,2-diméthyl 3-[(2,2-difluorocyclopropylidène) méthyl] cyclopropane carboxylate de (2,3,6-trifluorophényl) méthyle

rf = 0,2 (hexane-éther isopropylique 9-1)

### EXEMPLE 12 : [1R-[1alpha, 3alpha(E)]] 2,2-diméthyl 3-[(2,2-difluorocyclopropylidène) méthyl] cyclopropane carboxylate de (2,6-bis trifluorométhylphényl) méthyle

rf = 0,18 (hexane-éther isopropylique 95-5)

### EXEMPLE 13 : [1R-[1alpha, 3alpha(E)]] 2,2-diméthyl 3-[(2,2-difluorocyclopropylidène) méthyl] cyclopropane carboxylate de (1-(3-phénoxypyridyl) éthyle

rf = 0,1 (hexane-éther isopropylique 9-1)

### EXEMPLE 14 : [1R-[1alpha, 3alpha(E)]] 2,2-diméthyl 3-[(2,2-difluorocyclopropylidène) méthyl] cyclopropane carboxylate de (1-cyano (2-fluoro 3-phénoxyphényl) méthyle

rf = 0,09 (hexane-éther isopropylique 9-1)

### EXEMPLE 15 : [1R-[1alpha, 3alpha(E)]] 2,2-diméthyl 3-[(2,2-difluorocyclopropylidène) méthyl] cyclopropane carboxylate de (1-éthynyl (2-difluorométhylfuryl) méthyle

rf = 0,13 (hexane-éther isopropylique 9-1)

### EXEMPLE 16 : [1R-[1alpha, 3alpha(E)]] 2,2-diméthyl 3-[(2,2-difluorocyclopropylidène) méthyl] cyclopropane carboxylate de (2-fluoro 6-(trifluorométhyl) phényl) méthyle

rf = 0,18 (hexane-éther isopropylique 95-5)

### EXEMPLE 17 : [1R-[1alpha, 3alpha(E)]] 2,2-diméthyl 3-[(2,2-difluorocyclopropylidène) méthyl] cyclopropane carboxylate de (2-(difluorométhyl) furyl) méthyle

rf = 0,15 (hexane-éther isopropylique 9-1)

### EXEMPLE 18 : [1R-[1alpha, 3alpha(E)]] 2,2-diméthyl 3-[(2,2-difluorocyclopropylidène) méthyl] cyclopropane carboxylate de (1-(2-trifluorométhyl thiazol-4-yl) 2-propynyle

rf = 0,15 (hexane-chlorure de méthylène 7-3)

### EXEMPLE 19 : [1R-[1alpha, 3alpha(Z)]] 2,2-diméthyl 3-[(2,2-difluoro 3(R ou S)-n-butylcyclopropylidène) méthyl] cyclopropane carboxylate de (1-cyano (3-phénoxyphényl) méthyle

rf = 0,2 (hexane-éther isopropylique 9-1)

### EXEMPLE 20 : [1R-[1alpha, 3alpha(Z)]] 2,2-diméthyl 3-[(2,2-difluoro 3(R ou S)-n-butylcyclopropylidène) méthyl] cyclopropane carboxylate de (2,3,5,6-tétrafluorophényl) méthyle

rf = 0,1 (hexane-chlorure de méthylène 9-1)

### EXEMPLE 21 : [1R-[1alpha, 3alpha(Z)]] 2,2-diméthyl 3-[(2,2-difluoro 3(R ou S)-n-butylcyclopropylidène) méthyl] cyclopropane carboxylate de (4-propynyl 2,3,5,6-tétrafluorophényl) méthyle

rf = 0,12 (hexane-éther isopropylique 97-3)

### EXEMPLE 22 : [1R-[1alpha, 3alpha(E)]] 2,2-diméthyl 3-[(2,2-difluoro 3-méthylcyclopropylidène) méthyl] cyclopropane carboxylate de (2,3,5,6-tétrafluorophényl) méthyle

rf = 0,15 (hexane-chlorure de méthylène 8-2)

### EXEMPLE 23 : [1R-[1alpha, 3alpha(Z)]] 2,2-diméthyl 3-[(2,2-difluoro 3-méthylcyclopropylidène) méthyl] cyclopropane carboxylate de (2,3,5,6-tétrafluorophényl) méthyle

rf = 0,1 (hexane-chlorure de méthylène 8-2)

### EXEMPLE 24 : [1R-[1alpha, 3alpha(E)]] 2,2-diméthyl 3-[(2,2-difluoro 3-méthylcyclopropylidène) méthyl] cyclopropane carboxylate de (4-propynyl 2,3,5,6-tétrafluorophényl) méthyle

rf = 0,1 (hexane-éther isopropylique 97-3)

### EXEMPLE 25 : [1R-[1alpha, 3alpha(Z)]] 2,2-diméthyl 3-[(2,2-difluoro 3-méthylcyclopropylidène) méthyl] cyclopropane carboxylate de (4-propynyl 2,3,5,6-tétrafluorophényl) méthyle

rf = 0,15 (hexane-éther isopropylique 95-5)

### EXEMPLE 26 : [1R-[1alpha, 3alpha(E)]] 2,2-diméthyl 3-[(2,2-difluoro 3-méthylcyclopropylidène) méthyl] cyclopropane carboxylate de (1-cyano (3-phénoxyphényl) méthyle

rf = 0,1 (hexane-éther isopropylique 9-1)

### EXEMPLE 27 : [1R-[1alpha, 3alpha(Z)]] 2,2-diméthyl 3-[(2,2-difluoro 3-méthylcyclopropylidène) méthyl] cyclopropane carboxylate de (1-cyano (3-phénoxyphényl) méthyle

rf = 0,15 (hexane-éther isopropylique 9-1)

### EXEMPLE 28 : [1R-[1alpha, 3alpha(E)]] 2,2-diméthyl 3-[(2,2-difluoro 3-trifluorométhylclopropylidène) méthyl] cyclopropane carboxylate de 1-(2-trifluorométhylthiazol-4-yl) 2-propynyle

rf = 0,1 (hexane-éther isopropylique 95-5)

### PREPARATION 1 : Acide [1R-[1alpha, 3alpha(Z)]] 2,2-diméthyl 3-[2,2-difluorocyclopropylidène] méthyl cyclopropane carboxylique et isomères E et Z correspondants

### STADE A : le [1R-[1alpha, 3alpha(R)]] 2,2-diméthyl 3-[1-acétoxy propynyl) cyclopropane carboxylate de (1,1-diméthyl) éthyle.

On introduit à 0°C, en 15 minutes, 10,8 cm³ d'anhydride acétique dans une solution renfermant 8,5 g de [1R-[1alpha, 3alpha(R)]] 2,2-diméthyl 3-[1-hydroxy propynyl) cyclopropane carboxylate de (1,1-diméthyl) éthyle obtenu comme indiqué dans le brevet européen n° 0 105 006 et 25 cm³ de pyridine. On maintient le mélange réactionnel sous agitation pendant 6 heures, le verse sur une solution aqueuse de phosphate acide de sodium, et extrait à l'éther isopropylique. On sèche et amène à sec sous pression réduite. On chromatographie le produit obtenu sur silice en éluant avec le mélange hexane-éther isopropylique (7-3). On obtient 9,44 g de produit de rf = 0,4. F = 67,2°C.

### STADE B : [1R-[1alpha, 3alpha(R)]] 2,2-diméthyl 3-[1-acétoxy 1-(2,2-difluorocyclopropényl) éthyl] cyclopropane carboxylate de (1,1-diméthyl) éthyle

On introduit à 160°C, en 2 H 45 une solution renfermant 61,18 g de chlorodifluoroacétate de sodium et 230 cm³ de diglyme, dans une solution renfermant 10,7 g de produit obtenu au stade A, et 50 cm³ de diglyme. On maintient sous agitation pendant 1 heure, refroidit à 20°C, verse sur une solution aqueuse de phosphate acide de sodium, et extrait à l'éther. On sèche et amène à sec. On élimine le diglyme, en reprenant le produit au pentane. On lave à l'eau, sèche et amène à sec. On chromatographie le produit sur silice en éluant avec le mélange hexane-éther isopropylique (85-15). On obtient 8,57 g de produit recherché de rf = 0,2.

### STADE C : [1R-[1alpha, 3alpha(Z)]] 2,2-diméthyl 3-[2,2-difluorocyclopropylidène) méthyl] cyclopropane carboxylate de (1,1-diméthyl) éthyle, isomère E correspondant, isomère Z correspondant.

On ajoute à la température ambiante 26,6 g d'hydrure de triphénylphosphine de cuivre hexamère [Φ₃PCuH]₆ dans une solution renfermant 8,57 g de produit préparé au stade B et 170 cm³ de toluène. On maintient le mélange réactionnel sous agitation pendant 1 heure. On ajoute 80 cm³ de pentane et 80 cm³ d'éther éthylique. On agite pendant 2 heures, ajoute du clarcel agite à nouveau, filtre et amène à sec. On chromatographie le produit brut obtenu en éluant avec le mélange hexane-chlorure de méthylène (95-5). Après évaporation, sous pression réduite, on obtient :
720 mg de mélange E+Z
2,83 g d'isomère Z, rf = 0,15 (hexane-chlorure de méthylène 95-5)
2,97 g d'isomère E, rf = 0,1 ; F = 33,8°C.

### STADE D : Acide [1R-[1alpha, 3alpha(Z)]] 2,2-diméthyl 3-[(2,2-difluorocyclopropylidène) méthyl] cyclopropane carboxylique

On porte au reflux pendant 1 h 45 mn, une solution renfermant 2,83 g de produit isomère Z obtenu au stade C, 28 cm³ de toluène et 180 mg d'acide paratoluènesulfonique. On refroidit le mélange réactionnel à 20°C, verse sur de l'eau glacée, extrait à l'éther isopropylique, sèche et amène à sec sous pression réduite. On chromatographie le produit obtenu en éluant avec le mélange hexane-chlorure de méthylène-acétone (70-15-15). On évapore sous pression réduite et recueille 550 mg de produit recherché. rf = 0,15 (hexane-chlorure de méthylène-acétone 70-15-15).

### Acide [1R-[1alpha, 3alpha(E)]] 2,2-diméthyl 3-[(2,2-difluorocyclopropylidène) méthyl] cyclopropane carboxylique

En opérant comme pour la préparation de l'acide Z à partir de 2,97 g de l'ester de départ E, on a obtenu 1,88 g du produit recherché. rf = 0,15 (hexane-chlorure de méthylène-acétone 70-15-15) F = 78,9°C.

### Acide [1R-[1alpha, 3alpha(E+Z)]] 2,2-diméthyl 3-[(2,2-difluorocyclopropylidène) méthyl] cyclopropane carboxylique

En opérant comme pour la préparation de l'acide Z à partir de 720 mg de l'ester E+Z préparé précédemment, on a obtenu 320 mg du produit recherché. rf = 0,15 (hexane-chlorure de méthylène-acétone 70-15-15).

### PREPARATION 2 : Acide [1R-[1alpha, 3alpha(E+Z)]] 2,2-diméthyl 3-[2,2-difluoro 3-méthylcyclopropylidène] méthyl cyclopropane carboxylique et isomères E et Z correspondants

### STADE A : le [1R-[1alpha, 3alpha(E+Z)]] 2,2-diméthyl 3-[1-hydroxy 1-[2,2-(difluorocyclopropényl) éthyl] cyclopropane carboxylate de (1,1-diméthyl) éthyle.

On refroidit à 0°/+5°C 20 g d'acétate obtenu à la préparation 1B dans 200 cm³ de méthanol et ajoute en 45 minutes 63,5 cm³ d'une solution aqueuse de soude 1N. On agite 2 heures à 0°/+5°C, verse sur une solution aqueuse glacée de phosphate acide de sodium, extrait à l'éther, sèche et évapore le solvant. On obtient 17,24 g de produit attendu.

### STADE B : le [1R-[1alpha, 3alpha(E+Z)]] 2,2-diméthyl 3-[1-mésyl oxy 1-[2,2-(difluorocyclopropényl) éthyl] cyclopropane carboxylate de (1,1-diméthyl) éthyle.

On ajoute 4,95 cm³ de chlorure de mésyle à 16,9 g d'alcool préparé au stade A en solution dans 100 cm³ d'éther refroidi à 0°/+3°C. On agite puis ajoute en 30 minutes 8,95 cm³ de triéthylamine dans 35 cm³ d'éther. On agite 2 heures à 0°/+3°C, filtre, verse dans l'eau glacée, extrait à l'éther, lave à l'eau, sèche et évapore le solvant. On récupère 20,18 g de produit attendu.

### STADE C : le [1R-[1alpha, 3alpha(E+Z)]] 2,2-diméthyl 3-[2,2-difluoro 3(R ou S)-méthylcyclopropylidèneméthyl] cyclopropane carboxylate de (1,1-diméthyl) éthyle.

On refroidit à -20°/-25°C 10 g de cyanure de cuivre dans 100 cm³ de tétrahydrofuranne, ajoute 140 cm³ d'une solution de méthyllithium (1,6M) dans l'éther, agite 30 minutes à -15°C environ, verse dans une solution refroidie à -30°C comprenant 20,18 g de mésylate obtenu au stade B dans 200 cm³ de tétrahydrofuranne. On agite 30 minutes à -20°C, verse sur une solution aqueuse glacée de chlorure d'ammonium, extrait à l'éther, lave à l'eau, sèche et évapore le solvant. On chromatographie le résidu sur silice (éluant : hexane-chlorure de méthylène 9-1) et recueille 8,9 g de produit isomère Z et 2,44 g de l'isomère E correspondant.

### STADE D : Acide [1R-[1alpha, 3alpha(E+Z)]] 2,2-diméthyl 3[2,2-difluoro 3-méthylcyclopropylidène] méthyl cyclopropane carboxylique et isomères E et Z correspondants

On refroidit à 0°/+2°C 4 g d'isomère Z obtenu au stade C dans 40 cm³ de chlorure de méthylène, ajoute 11 cm³ d'acide trifluoroacétique et agite 3 heures à 0°/+2°C. On verse le milieu réactionnel dans l'eau galcée, extrait au chlorure de méthylène, lave à l'eau, sèche, évapore le solvant, chromatographie le résidu sur silice (éluant : hexane-acétone-chlorure de méthylène 70-15-15) et recueille 2,11 g de produit attendu sous forme d'isomère Z. F = 53°C.

En opérant de la même manière au départ de 2,44 g de l'ester isomère E obtenu au stade C, on obtient 1,31 g de produit attendu sous forme d'isomère E. F = 97,7°C.

### PREPARATION 3 : Acide [1R-[1alpha, 3alpha(E+Z)]] 2,2-diméthyl 3-[2,2-difluoro 3-terbutylcyclopropylidène] méthyl cyclopropane carboxylique et isomères E et Z correspondants.

On opère comme à la préparation 2 en utilisant au départ l'acétate obtenu au stade B de la préparation 1 et du terbutyllithium.

### PREPARATION 4 : Acide [1R-[1alpha, 3alpha(E+Z)]] 2,2-diméthyl 3-[2,2-difluoro 3-trifluorométhylcyclopropylidène] méthyl cyclopropane carboxylique et isomères E et Z correspondants.

### STADE A : [1R-[1alpha, 3alpha(R)]] 2,2-diméthyl 3-(1-hydroxy 3-trifluoro 2-butynyl) cyclopropane carboxylate de (1,1-diméthyl) éthyle.

On mélange 1,75 cm³ de butyllithium (1,5M) dans 5 cm³ de tétrahydrofuranne à -40°C, fait réagir du trifluoro 1-propynyl gazeux (2,5M), ajoute en 20 minutes à -60°C 500 mg de 2,2-diméthyl 3-formyl cyclopropane carboxylate de (1,1-diméthyl) éthyle, agite 30 minutes, verse sur une solution aqueuse saturée de phosphate acide de sodium, extrait à l'éther, sèche, évapore le solvant sous pression réduite, chromatographie le résidu sur silice (éluant : hexane-acétate d'éthyle 9-1) et obtient 400 mg de produit attendu. F = 45,7°C.

### STADE B : [1R-[1alpha, 3alpha(R)]] 2,2-diméthyl 3-(1-acétoxy 3-trifluoro 2-butynyl) cyclopropane carboxylate de (1,1-diméthyl) éthyle.

On opère comme au stade A de la préparation 1 en utilisant au départ 400 mg de produit obtenu ci-dessus et 0,48 cm³ d'anhydride acétique. On obtient 410 mg de produit attendu.

### STADE C : [1R-[1alpha, 3alpha(R)]] 2,2-diméthyl 3-[(1-acétoxy 1-(2,2-difluoro 3-trifluorométhyl cyclopropényl) éthyl] cyclopropane carboxylate de (1,1-diméthyl) éthyle.

On opère comme à la préparation 1 stade B au départ de 410 mg de produit obtenu ci-dessus au stade B et 1,97 g de chlorodifluoroacétate de sodium. On obtient 310 mg de produit attendu.

### STADE D : [1R-[1alpha, 3alpha(E,Z)]] 2,2-diméthyl 3-[2,2-difluoro 3-trifluorométhylcyclopropylidène) méthyl] cyclopropane carboxylate de (1,1-diméthyl) éthyle et ses isomères E et Z correspondants.

On refroidit à -60°/-70°C, 3,28 g de produit obtenu au stade C dans 50 cm³ d'hexane et 5 cm³ de tétrahydrofuranne, ajoute goutte à goutte en 10 minutes 8,5 cm³ de borohydrure de tributyle potassium. On agite 10 minutes à -60°C, verse sur une solution aqueuse glacée de phosphate acide de potassium, extrait à l'éther, sèche et évapore le solvant sous pression réduite et obtient 4 g de produit attendu.

### STADE E : Acide [1R-[1alpha, 3alpha(E+Z)]] 2,2-diméthyl 3-[2,2-difluoro 3-trifluorométhylcyclopropylidène] méthyl cyclopropane carboxylique et isomères E et Z correspondants.

On refroidit à -3°C le produit obtenu au stade précédent dans 26 cm³ de chlorure de méthylène, ajoute en 5 minutes 6,6 cm³ d'acide trifluoroacétique, agite 30 minutes à +10°C puis 2 heures à 20°C. On verse le milieu réactionnel dans l'eau glacée, extrait au chlorure de méthylène, sèche, évapore le solvant, chromatographie le résidu sur silice (éluant : hexane-acétate d'éthyle 1-1) et obtient 1,8 g de produit attendu.

### PREPARATION 5 : Alcool 2,3,6-trifluoro benzylique

On dissout à la température ambiante, sous atmosphère d'azote 10,56 g d'acide 2,3,6-trifluoro benzoïque dans 100 cm³ de tétrahydrofuranne. On refroidit au bain de glace-méthanol et ajoute en 30 minutes une solution renfermant 10 cm³ de complexe borane méthyl sulfure à 10 millimoles/cm³ et 30 cm³ de tétrahydrofuranne. On agite encore pendant 5 minutes et laisse revenir à la température ambiante. On chauffe pendant 3 h 30 à 45°/50°C. On verse la solution obtenue sur une solution aqueuse de phosphate acide de sodium. On extrait à l'éther isopropylique, lave, sèche et amène à sec. On obtient 10,6 g de liquide incolore que l'on chromatographie sur silice en éluant avec le mélange hexane-acétate d'éthyle (7-3). On obtient 8,8 g de produit recherché. F < 50°C.
Spectre IR (CHCl₃)
- OH: 3620 cm⁻¹
- Aromatique: 1642, 1604, 1499 cm⁻¹

Spectre de RMN (CDCl₃, 60 Hz) ppm
- Protons aromatiques: 6,70 - 7,47
- CH₂-OH: 4,83
- OH: 2,20

### PREPARATION 6 : Alcool 2,6-bis(trifluorométhyl) benzylique

### STADE A : 2,6-bis(trifluorométhyl) benzoate de méthyle

On agite pendant 30 minutes à 20°C, une solution renfermant 6 g d'acide 2,6-bis(trifluorométhyl) benzoïque, 60 ml de tétrahydrofuranne et 11,58 ml d'une solution de soude 2N. On refroidit à 0°C et ajoute 4,26 ml de sulfate de diméthyle. On agite 1 heure à 20°C et ajoute à nouveau 2,1 ml de sulfate de diméthyle et maintient le mélange réactionnel sous agitation pendant 24 heures à 20°C. On le verse sur une solution aqueuse de bicarbonate de sodium, on extrait à l'éther isopropylique puis à l'acétate d'éthyle. On sèche, filtre, rince et amène à sec. On obtient ainsi, après chromatographie sur silice (éluant hexane-acétate d'éthyle (9-1)) 5,59 g du produit recherché.

### STADE B : Alcool 2,6-bis(trifluorométhyl) benzylique

On ajoute à 0°C, 58 ml d'une solution 1,2 M d'hydrure de diisobutylaluminium (DIBAH) dans une solution renfermant 5,59 g du produit préparé au stade A et 60 ml de toluène. On laisse la température revenir à 20°C et maintient le mélange réactionnel sous agitation pendant 4 heures. On verse dans une solution molaire de tartrate double de potassium et de sodium. On extrait à l'éther isopropylique, sature la phase aqueuse avec du chlorure de sodium. On extrait à l'acétate d'éthyle, sèche, filtre, rince et amène à sec. Après chromatographie sur silice (éluant : hexane-acétate d'éthyle (9-1)) on obtient 4,72 g de produit recherché.

### PREPARATION 7 : 5-(difluorométhyl)-2-furanméthanol.

### STADE A : 5-(acétyloxy) méthyl-2-furancarboxaldéhyde.

On ajoute à 5°C, 10,05 cm³ de chlorure d'acétyle dans une solution renfermant 16,2 g de 5-(hydroxy méthyl) 2-furancarboxaldéhyde et 200 cm³ de chlorure de méthylène. On ajoute ensuite 11,4 cm³ de pyridine et 50 cm³ de chlorure de méthylène. On maintient le mélange réactionnel sous agitation pendant 3 heures à 20°C. On traite avec une solution aqueuse de phosphate acide de sodium et extrait au chlorure de méthylène. On sèche et concentre. On obtient après chromatographie sur silice en éluant avec le mélange hexane-acétate d'éthyle (7-3), 19,35 g de produit recherché.

### STADE B : Acétate de 5-(difluorométhyl)-2-furan méthanol.

On ajoute à 5°C une solution renfermant 7,26 cm³ de DAST (diéthylamino sulfure trifluorure) et 30 cm³ de chlorure de méthylène, dans une solution renfermant 10 g de produit préparé au stade A et 100 cm³ de chlorure de méthylène. On agite le mélange réactionnel à 20°C pendant une demi heure et porte au reflux pendant 1 heure. On maintient sous agitation pendant 18 heures à 20°C et porte à reflux pendant 3 heures. On traite avec du carbonate acide de sodium et extrait au chlorure de méthylène. On sèche et concentre. On obtient 12 g d'un produit que l'on chromatographie sur silice en éluant avec le mélange hexane-acétate d'éthyle 8-2. On obtient ainsi 5,5 g du produit recherché.

### STADE C : 5-(difluoro méthyl)-2-furanméthanol.

On ajoute 34,8 cm³ d'une solution normale de soude dans une solution renfermant 5,5 g de produit préparé au stade précédent et 100 cm³ de méthanol. On agite pendant 1 heure à 20°C. On traite avec une solution saturée de phosphate acide de sodium. On concentre et chromatographie sur silice le produit obtenu en éluant avec le mélange hexane-acétate d'éthyle 7-3. On obtient ainsi 3,6 g de produit recherché.

### PREPARATION 8 : 5-(difluorométhyl) alpha-éthynyl 2-furanméthanol.

### STADE A : 5-difluorométhyl 2-furancarboxaldéhyde.

On ajoute à -60°C, sous atmosphère d'azote une solution renfermant 6,5 cm³ de DMSO (diméthyl sulfoxyde) et 60 cm³ de chlorure de méthylène dans une solution renfermant 3,84 cm³ de chlorure d'oxalyle et 40 cm³ de chlorure de méthylène. On maintient le mélange réactionnel sous agitation à -60°C et ajoute 3,6 g de 5-difluorométhyl 2-furanméthanol en solution dans 30 cm³ de chlorure de méthylène. On agite pendant 2 heures à -60°C et ajoute en un quart d'heure une solution de 16,1 cm³ de triéthylamine et 30 cm³ de chlorure de méthylène. On maintient le mélange réactionnel sous agitation pendant une demi heure à -60°C, laisse la température remonter à -20°C et agite pendant une demi heure à cette température. On verse sur une solution de phosphate acide de sodium, extrait au chlorure de méthylène et sèche. On obtient 3,6 g de produit que l'on chromatographie sur silice en éluant avec le mélange hexane-acétate d'éthyle 8-2. On obtient après chromatographie 2,5 g de produit recherché.

### STADE B : [5-difluorométhyl 2-furyl] 2-propynol.

On ajoute à 0°C en une heure 43 cm³ d'une solution 0,5 M de bromure d'éthynyl magnésien dans le tétrahydrofuranne, dans une solution renfermant 2,5 g de produit préparé au stade A et 30 cm³ de tétrahydrofuranne. On maintient le mélange réactionnel sous agitation à 0°C pendant 1 heure. On verse sur une solution aqueuse saturée en phosphate acide de sodium. On obtient 3 g de produit que l'on chromatographie sur silice en éluant avec le mélange hexane-acétate d'éthyle 7-3. On obtient 2,5 g de produit recherché.

### PREPARATION 9 : alpha-éthynyl 2-(trifluorométhyl) 4-thiazolyl méthanol.

On ajoute 11 cm³ d'une solution molaire de bromure d'éthynyl magnésium dans une solution de tétrahydrofuranne renfermant 2 g de 2-(trifluorométhyl) 4-thiazolecarboxaldéhyde. On maintient le mélange réactionnel sous agitation pendant 30 minutes à 20 ≃ 25°C. On verse sur une solution de chlorure d'ammonium. On extrait au chlorure de méthylène, sèche, filtre et amène à sec. On obtient 2,1 g de produit recherché.

### EXEMPLE 29 : Préparation d'un concentré soluble

On effectue un mélange homogène de :

| | |
|---|---|
| Produit de l'exemple 2 | : 0,25 g |
| Butoxyde pipéronyle | : 1,00 g |
| Tween 80 | : 0,25 |
| Topanol A | : 0,1 g |
| Eau | : 98,4 g |

### EXEMPLE 30 : Préparation d'un concentré émulsifiable

On mélange intimement :

| | |
|---|---|
| Produit de l'exemple 4 | : 0,015 g |
| Butoxyde de pipéronyle | : 0,5 g |
| Topanol A | : 0,1 g |
| Tween 80 | : 3,5 g |
| Xylène | : 95,885 g |

### EXEMPLE 31 : Préparation d'un concentré émulsifiable

On effectue un mélange homogène de :

| | |
|---|---|
| Produit de l'exemple 2 | : 1,5 g |
| Tween 80 | : 20,00 g |
| Topanol A | : 0,1 g |
| Xylène | : 78,4 g |

### EXEMPLE 32 : Préparation d'une composition fumigène

On mélange d'une façon homogène :

| | |
|---|---|
| Produit de l'exemple 4 | : 0,25 g |
| Poudre de tabu | : 25,00 g |
| Poudre de feuille de cèdre | : 40,00 g |
| Poudre de bois de pin | : 33,75 g |
| Vert brillant | : 0,5 g |
| p-Nitrophénol | : 0,5 g |

### ETUDE BIOLOGIQUE

### A) Etude de l'effet létal sur larves de Spodoptera Littoralis

Les essais sont effectués par application topique d'une solution acétonique à l'aide du micro manipulateur d'Arnold sur le thorax dorsal des larves. On utilise 15 larves par dose de produit à tester. Les larves utilisées sont des larves du quatrième stade larvaire, c'est-à-dire âgées d'environ 10 jours lorsqu'elles sont élevées à 24°C et 65 % d'humidité relative. Après traitement les individus sont placés sur milieu nutritif artificiel (milieu de Poitout).

On effectue le contrôle des mortalités 48 heures après traitement.

A la dose de 10 ppm, les produits des exemples 6, 13, 14 et 27 présentent une bonne activité.

### B) Etude de l'activité sur Diabrotica

Les insectes tests sont des larves de dernier stade de Diabrotica.

On traite une rondelle de papier filtre de 9 cm de diamètre, déposée au fond d'une boîte de pétri, à l'aide de 2 cm³ de solution acétonique. Après séchage on dépose 10 larves par dose et on effectue le contrôle de mortalité 24 h après le traitement.

A la dose de 10 ppm, les produits des exemples 2, 4, 7, 8, 11, 12, 15, 16, 18 et 22 présentent une bonne activité.

### C) Etude de l'effet létal sur Aphis cracivora.

On utilise des adultes après 7 jours et l'on emploie 10 Aphis par concentration utilisée. On utilise une méthode de contact-ingestion. On effectue le traitement au pistolet de Fisher d'une feuille de fève que l'on dépose dans une boîte de pétri en matière plastique sur une rondelle de papier humidifiée. Le traitement est effectué à l'aide de 2 cm³ de solution acétonique de produit à tester (1 cm³ par face de feuille). L'infestation par insecte est effectuée après séchage de la feuille. On maintient les insectes en contact avec la feuille pendant une heure. On place les insectes sur des feuilles non traitées et contrôle la mortalité au bout de 24 heures.
Résultats : A la dose de 10 ppm les produits des exemples 3, 4, 6, 8, 13, 14, 19, 21, 24, 25, 26 et 27 présentent une bonne activité.

## Revendications

**1)** Sous toutes leurs formes stéréoisomères possibles, ainsi que leurs mélanges les composés de formule (I) : 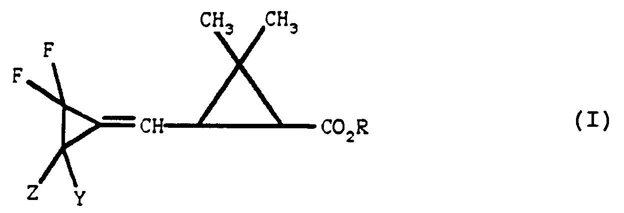 dans lesquels :
- Y et Z, identiques ou différents l'un de l'autre, représentent un atome d'hydrogène, un atome d'halogène, un radical CF₃, un radical alkyle renfermant jusqu'à 8 atomes de carbone, un radical O-alkyle, renfermant jusqu'à 8 atomes de carbone, ou un radical S-alkyle renfermant jusqu'à 8 atomes de carbone, ou un radical aryle, O-aryle ou S-aryle renfermant jusqu'à 14 atomes de carbone éventuellement substitué par un ou plusieurs atomes d'halogène, un ou plusieurs radicaux hydroxyle estérifiés ou éthérifiés, un ou plusieurs radicaux CF₃, un ou plusieurs radicaux alkyle renfermant jusqu'à 8 atomes de carbone,
- R représente un radical alkyle renfermant jusqu'à 8 atomes de carbone, ou le reste d'un alcool utilisé dans la synthèse des esters pyréthrinoïdes,
sous toutes leurs formes stéréoisomères possibles ainsi que leurs mélanges.

**2)** Les composés de formule (I) tels que définis à la revendication 1 dans lesquels Y représente un atome d'hydrogène.

**3)** Les composés de formule (I) tels que définis à la revendication 2 dans lesquels Z représente un atome d'hydrogène.

**4)** Les composés de formule (I) tels que définis à la revendication 1, 2 ou 3 dans lesquels la copule 2,2-diméthyl cyclopropanique est de structure 1R cis.

**5)** Les composés de formule (I) tels que définis à la revendication 1, 2, 3 ou 4 dans lesquels la géométrie de la double liaison est E.

**6)** Les composés de formule (I) tels que définis à l'une quelconque des revendications 1 à 5 dans lesquels R représente :
- soit un radical alkyle, renfermant jusqu'à 8 atomes de carbone,
- soit un radical benzyle éventuellement substitué par un ou plusieurs radicaux choisis dans le groupe constitué par les radicaux alkyles comportant de 1 à 4 atomes de carbone, éventuellement substitués par un ou plusieurs atomes d'halogène, les radicaux alkyloxyles comportant de 1 à 4 atomes de carbone éventuellement substitués par un ou plusieurs atomes d'halogène, les radicaux alcényles comportant de 2 à 6 atomes de carbone, les radicaux alcényloxy comportant de 2 à 6 atomes de carbone, les radicaux alcadiényles comportant de 4 à 8 atomes de carbone, le radical méthylènedioxy et les atomes d'halogène,
- soit un groupement : 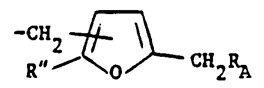 dans lequel le substituant R'' représente un atome d'hydrogène ou un radical méthyle et le substituant R_{A} un aryle monocyclique ou un groupement -C≡CH et notamment un groupement 5-benzyl 3-furyl méthyle,
- soit un groupement : 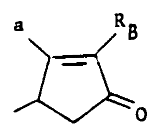 dans lequel a représente un atome d'hydrogène ou un radical méthyle et R_{B} représente un radical organique aliphatique comportant de 2 à 6 atomes de carbone et une ou plusieurs insaturations carbone-carbone et notamment le radical -CH₂-CH=CH₂, -CH₂-CH=CH-CH₃, -CH₂-CH=CH-CH=CH₂, -CH₂-CH=CH-CH₂-CH₃, -CH₂-C≡CH,
- soit un groupement : 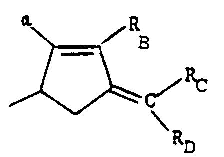 dans lequel a et R_{B} conservent la même signification que précédemment, R_{C} et R_{D}, identiques ou différents, représentent un atome d'hydrogène, un atome d'halogène, un radical alkyle renfermant de 1 à 6 atomes de carbone, un radical aryle comportant de 6 à 10 atomes de carbone, un groupement alkyloxycarbonyle comportant de 2 à 5 atomes de carbone, ou un groupement cyano,
- soit un groupement : 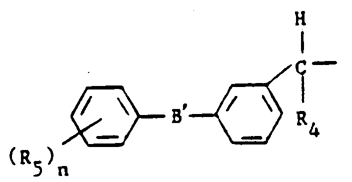 dans lequel B' représente un atome d'oxygène ou de soufre, un groupement 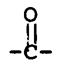 ou -CH₂ ou un groupement sulfoxyde ou un groupement sulfone et R₄ représente un atome d'hydrogène, un radical -C≡N, un radical méthyle, un radical -CONH₂, un radical -CSNH₂ ou un radical -C≡CH, R₅ représente un atome d'hydrogène, un atome d'halogène ou un radical méthyle et n représente un nombre égal à 0,1 ou 2, et notamment le groupement 3-phénoxy benzyle, alpha-cyano 3-phénoxy benzyle, alpha-éthynyl 3-phénoxy benzyle, 3-benzoyl benzyle, 1-(3-phénoxy phényl) éthyle ou alpha-thioamido 3-phénoxy benzyle,
- soit un groupement : 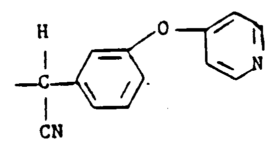
- soit un groupement : 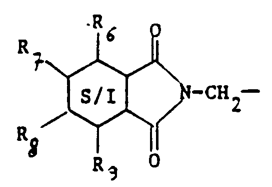 dans lequel les substituants R₆, R₇, R₈, R₉ représentent un atome d'hydrogène, un atome de chlore, ou un radical méthyle et dans lequel S/I symbolise un cycle aromatique ou un cycle analogue dihydro ou tétrahydro,
- soit un groupement : 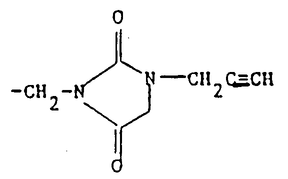
- soit un groupement : 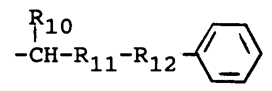 dans lequel R₁₀ représente un atome d'hydrogène ou un radical CN, R₁₂ représente un radical -CH₂- ou un atome d'oxygène, R₁₁ représente un radical thiazolyle ou thiadiazolyle dont la liaison avec 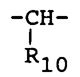 peut se trouver à l'une quelconque des positions disponibles, R₁₂ étant lié à R₁₁ par l'atome de carbone compris entre l'atome de soufre et un atome d'azote,
- soit un groupement : 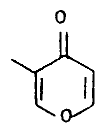
- soit un groupement : 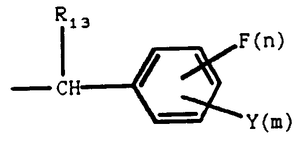 dans lequel R₁₃ représente un atome d'hydrogène, un radical CH₃, C≡N ou C≡CH,
n représente un nombre entier pouvant varier de 1 à 5,
m représente le nombre 5-n et,
- Y représente un atome d'hydrogène, un atome d'halogène, un radical CH₂-C≡N, un radical hydroxy, un radical alkyle linéaire, ramifié, saturé ou insaturé éventuellement substitué renfermant jusqu'à 8 atomes de carbone un radical nitrile, un radical 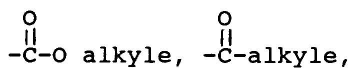 -(CH₂)m' O-alkyle, -(CH₂)m' S-alkyle, -(CH₂)m' N-(alkyle)₂, dans lequel m' représente le nombre 0, 1, 2, 3 ou 4 et renfermant jusqu'à 12 atomes de carbone, un radical Si (alkyle)₃, alkyle représente un radical alkyle linéaire ou ramifié, saturé ou insaturé, éventuellement substitué renfermant jusqu'à 8 atomes de carbone, un radical O-aryle ou -(CH₂)m'-aryle, aryle représentant un radical aryle renfermant jusqu'à 14 atomes de carbone, le ou les substituants Y identiques ou différents, pouvant être en position quelconque sur le noyau phényle,
- soit un groupement : 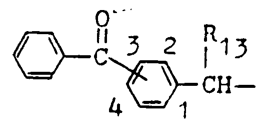 dans lequel R₁₃ est défini comme ci-dessus, et le radical benzoyle est lié aux positions 3 ou 4 du phényle,
- soit un groupement : 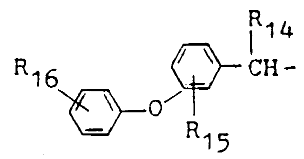 dans lequel R₁₄ représente un atome d'hydrogène, un radical méthyle, éthynyle ou cyano et R₁₅ et R₁₆, différents, représentent un atome d'hydrogène, de fluor ou de brome,
- soit un groupement : 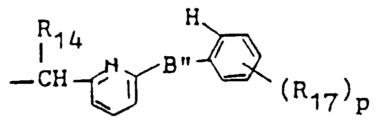 dans lequel R₁₄ est défini comme ci-dessus, chacun des R₁₇ représente indépendamment un atome d'hydrogène, un groupement alkyle renfermant de 1 à 4 atomes de carbone, alcoxy renfermant de 1 à 4 atomes de carbone, alkylthio renfermant de 1 à 4 atomes de carbone, alkylsulfonyl renfermant de 1 à 4 atomes de carbone, trifluorométhyl, 3,4-méthylènedioxy, chloro, fluoro ou bromo, p représente un nombre égal à 0,1 ou 2 et B'' représente un atome d'oxygène ou un atome de soufre,
- soit un groupement : 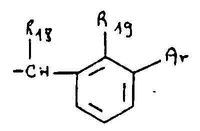 dans lequel R₁₈ représente un atome d'hydrogène, un radical méthyle, éthynyle ou cyano et R₁₉, différent, représente un atome d'hydrogène, de fluor ou de brome et Ar représente un radical aryle renfermant jusqu'à 14 atomes de carbone,
- soit un groupement 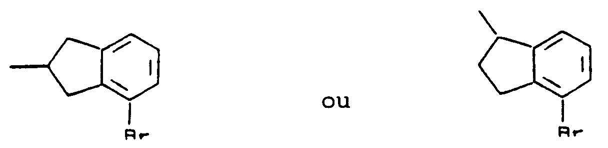 dans lesquels Ar est défini comme précédemment,
- soit un groupement : 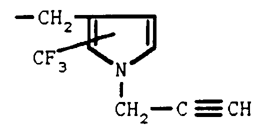 dans lequel le radical CF₃ est en position quelconque sur le noyau pyrrolique,
- soit un groupement 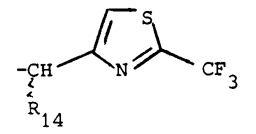 dans lequel R₁₄ est défini comme ci-dessus,
- soit un groupement 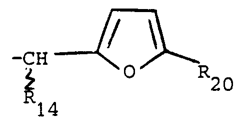 dans lequel R₁₄ est défini comme ci-dessus et R₂₀ représente un radical alkyle comportant de 1 à 4 atomes de carbone éventuellement substitué par un ou plusieurs atomes d'halogène.

**7)** Les composés de formule (I) tels que définis à la revendication 6 dans lesquels R représente un radical :

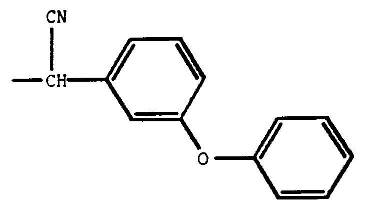

**8)** Les composés de formule (I) tels que définis à la revendication 6 dans lesquels R représente un radical : 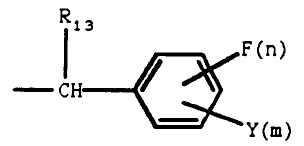 dans lequel R₁₃, m, n et Y sont définis comme précédemment.

**9)** Les composés de formule (I) tels que définis à la revendication 6 dans lesquels R représente un radical :

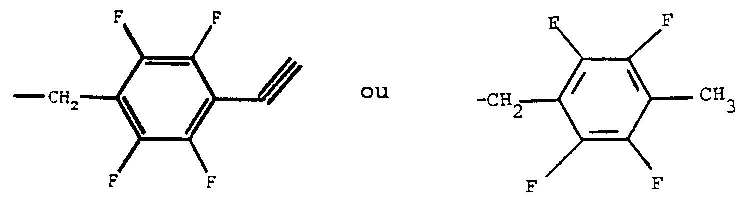

**10)** Les composés de formule (I) tels que définis à la revendication 6 dans lesquels R représente un radical :

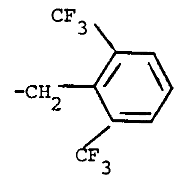

**11)** Les composés de formule (I) tels que définis à la revendication 6 dans lesquels R représente un radical : 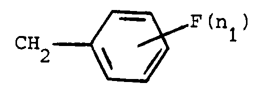 dans lequel n₁ = 3, 4 ou 5.

**12)** Les composés de formule (I) tels que définis à la revendication 6 dans lesquels R représente un radical :

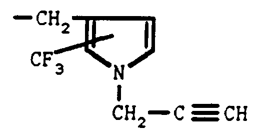

**13)** Les composés de formule (I) tels que définis à la revendication 6 dans lesquels R représente un radical : 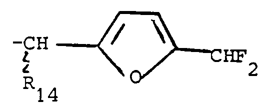 dans lequel R₁₄ représente un atome d'hydrogène ou un radical éthynyle.

**14)** Les composés de formule (I) tels que définis à la revendication 6 dans lesquels R représente un radical :

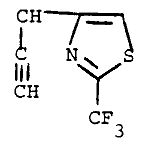

**15)** Les composés de formule (I) tels que définis à la revendication 1 dont les noms suivent :
- le [1R-[1alpha, 3alpha(E)]] 2,2-diméthyl 3-[(2,2-difluorocyclopropylidène) méthyl] cyclopropane carboxylate de (2,3,5,6-tétrafluorophényl) méthyle,
- le [1R-[1alpha, 3alpha(E)]] 2,2-diméthyl 3-[(2,2-difluorocyclopropylidène) méthyl] cyclopropane carboxylate de (2,3,5,6-tétrafluoro 4-propynyl phényl) méthyle,
- le [1R-[1alpha(S), 3alpha(E,Z)]] 2,2-diméthyl 3-[(2,2-difluorocyclopropylidène) méthyl] cyclopropane carboxylate de (1-cyano 3-phénoxy phényl) méthyle,
- le 1R-[1alpha, 3alpha(E,Z)] 2,2-diméthyl 3-[(2,2-difluoro cyclopropylidène) méthyl] cyclopropane carboxylate de (4-méthyl 2,3,5,6-tétrafluorophényl) méthyle,
- le [1R-[1alpha, 3alpha(E)]] 2,2-diméthyl 3-[(2,2-difluorocyclopropylidène) méthyl] cyclopropane carboxylate de (pentafluorophényl) méthyle
- le [1R-[1alpha, 3alpha(E)]] 2,2-diméthyl 3-[(2,2-difluorocyclopropylidène) méthyl] cyclopropane carboxylate de (2,3,6-trifluorophényl) méthyle
- le [1R-[1alpha, 3alpha(E)]] 2,2-diméthyl 3-[(2,2-difluorocyclopropylidène) méthyl] cyclopropane carboxylate de (2,6-bis trifluorométhylphényl) méthyle
- le [1R-[1alpha, 3alpha(E)]] 2,2-diméthyl 3-[(2,2-difluorocyclopropylidène) méthyl] cyclopropane carboxylate de (1-éthynyl (2-difluorométhylfuryl) méthyle
- le [1R-[1alpha, 3alpha(E)]] 2,2-diméthyl 3-[(2,2-difluorocyclopropylidène) méthyl] cyclopropane carboxylate de (2-fluoro 6-(trifluorométhyl) phényl) méthyle
- le [1R-[1alpha, 3alpha(E)]] 2,2-diméthyl 3-[(2,2-difluorocyclopropylidène) méthyl] cyclopropane carboxylate de (1-(2-trifluorométhyl thiazol-4-yl) 2-propynyle
- le [1R-[1alpha, 3alpha(E)]] 2,2-diméthyl 3-[(2,2-difluoro 3-méthylcyclopropylidène) méthyl] cyclopropane carboxylate de (2,3,5,6-tétrafluorophényl) méthyle,
ainsi que leurs diastéréoisomères et les mélanges de ces diastéréoisomères.

**16)** Application des composés de formule (I) tels que définis à l'une quelconque des revendications 1 à 15, à la lutte contre les parasites des végétaux et les parasites des locaux.

**17)** Les compositions destinées à la lutte contre les parasites des végétaux, les parasites des locaux et les parasites des animaux à sang chaud, caractérisées en ce qu'elles renferment comme principe actif au moins un composé défini à l'une quelconque des revendications 1 à 15.

**18)** Les compositions insecticides, caractérisées en ce qu'elles renferment comme principe actif au moins un composé défini à l'une quelconque des revendications 1 à 15.

**19)** Les compositions insecticides définies à la revendication 18 caractérisées en ce qu'elles sont destinées à la lutte contre DIABROTICA et les autres parasites du sol.

**20)** Les compositions acaricides, caractérisées en ce qu'elles renferment comme principe actif au moins un composé défini à l'une quelconque des revendications 1 à 15.

**21)** Associations douées d'activité insecticide, acaricide ou nématicide, caractérisées en ce qu'elles contiennent comme matière active, d'une part, un au moins des composés de formule générale (I) et d'autre part, un au moins des esters pyréthrinoïdes choisis dans le groupe constitué par les esters d'alléthrolones, d'alcool 3,4,5,6-tétrahydrophtalimido méthylique, d'alcool 5-benzyl 3-furyl méthylique, d'alcool 3-phénoxy benzylique et d'alcools alpha-cyano 3-phénoxy benzyliques des acides chrysanthémiques, par les esters d'alcool 5-benzyl 3-furyl méthylique des acides 2,2-diméthyl 3-(2-oxo 3-tétrahydrothiophénylidène méthyl) cyclopropane-1-carboxyliques, par les esters d'alcool 3-phénoxy benzylique et d'alcools alpha-cyano 3-phénoxy benzyliques des acides 2,2-diméthyl 3-(2,2-dichlorovinyl) cyclopropane-1-carboxyliques, par les esters d'alcools alpha-cyano 3-phénoxy benzyliques d'acides 2,2-diméthyl 3-(2,2-dibromovinyl) cyclopropane-1-carboxyliques, par les esters d'alcool 3-phénoxy benzylique des acides 2-parachlorophényl 2-isopropyl acétiques, par les esters d'alléthrolone, d'alcool 3,4,5,6-tétrahydrophtalimidométhylique, d'alcool 5-benzyl 3-furyl méthylique, d'alcool 3-phénoxy benzylique et d'alcool alphacyano 3-phénoxy benzylique des acides 2,2-diméthyl 3-(1,2,2,2-tétrahaloéthyl) cyclopropane-1-carboxyliques, dans lesquels "halo" représente un atome de fluor, de chlore ou de brome, étant entendu que les composés (I) peuvent exister sous toutes leurs formes stéréoisomères possibles de même que les copules acides et alcools des esters pyréthrinoïdes ci-dessus.

**22)** Procédé de préparation des composés de formule (I) tels que défini à l'une quelconque des revendications 1 à 15, caractérisé en ce que l'on soumet un acide de formule (II) : 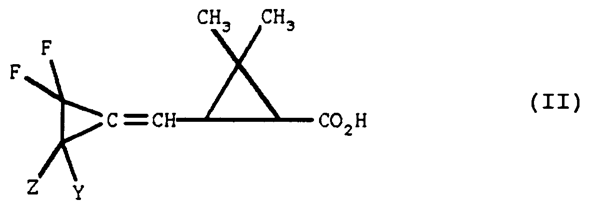 dans lequel Y et Z sont définis comme précédemment, ou un dérivé fonctionnel de cet acide, à l'action d'un alcool de formule (III) :
ROH (III)
R étant défini comme précédemment, pour obtenir le composé de formule (I) correspondant.

**23)** A titre de produits chimiques nouveaux, les acides de formule (II) : 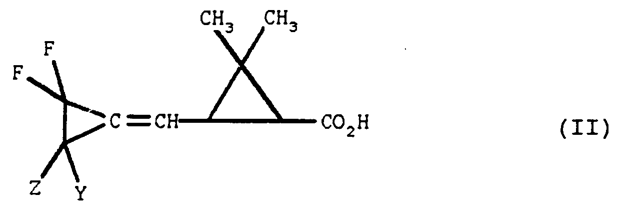 définie à la revendication 22, dans laquelle Y et Z sont définis comme précédemment.

**24)** A titre de produits chimiques nouveaux les acides de formule (II) tels que définis à la revendication 23, dans lesquels Y et Z représentent un atome d'hydrogène.

**25)** A titre de produits chimiques nouveaux :
- le 1R-[1alpha, 3alpha(Z)] 2,2-diméthyl 3-[(2,2-difluorocyclopropylidène) méthyl] cyclopropane carboxylate de (1,1-diméthyl) éthyle, l'isomère E correspondant ainsi que le mélange d'isomère E + Z correspondant.
